(19) Europäisches Patentamt
European Patent Office
Office européen des brevets

(11) Publication number: **0 602 654 A1**

# (12) EUROPEAN PATENT APPLICATION

(21) Application number: **93120340.0**

(22) Date of filing: **16.12.93**

(51) Int. Cl.5: **G02F 1/35**, C07D 409/06, C07D 409/14, C07D 417/06, C07D 495/04

(30) Priority: **18.12.92 US 992625**

(43) Date of publication of application: **22.06.94 Bulletin 94/25**

(84) Designated Contracting States: **AT BE CH DE DK ES FR GB GR IE IT LI LU MC NL PT SE**

(71) Applicant: **ENICHEM S.p.A.**
**Piazza della Repubblica, 16**
**I-20124 Milano(IT)**

(72) Inventor: **Jen, Kwan-Yue Alex**
**8 Schindler Drive**
**North Old Bridge, New Jersey 08857(US)**
Inventor: **Drost, Kevin Joel**
**23 Chestnut Street**
**Spotswood, New Jersey 08884(US)**
Inventor: **Wong, King Young**
**6010 Shadow Oaks Court**
**Monmouth Junction, New Jersey 08852(US)**
Inventor: **Varanasi, Pushkara Rao**
**1207 Sweetbriar Street**
**Monmouth Junction, New Jersey 08536(US)**
Inventor: **Mininni, Robert Mark**
**39 Sourland Hills Road**
**Skillman, New Jersey 08558(US)**

(74) Representative: **Weinhold, Peter, Dr. et al**
**Patentanwälte Dipl.-Ing. G. Dannenberg**
**Dr. P. Weinhold**
**Dr. D. Gudel**
**Dipl.-Ing. S. Schubert**
**Dr. P. Barz**
**Siegfriedstrasse 8**
**D-80803 München (DE)**

## (54) Efficient electron-donating groups for nonlinear optical applictions.

(57) Nonlinear optical materials having structures with delocalized resonance configurations corresponding to:

EP 0 602 654 A1

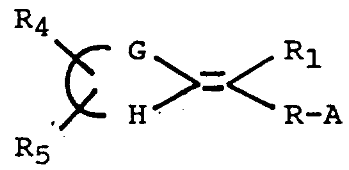

or:

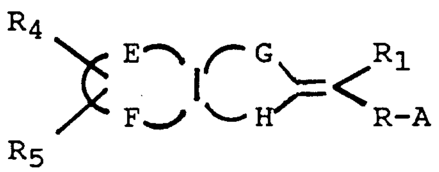

wherein A is a first electron accepting group, R is pi-conjugated non-centrosymmetric organic moiety; $R_4$ and $R_5$ are independently selected from hydrogen, alkyl moieties and functionalized alkyl moieties; E, F, G and H are members of a saturated or unsaturated five- to ten-membered cyclic ring or two-ring systems having five- to ten-membered rings that are electron donating in nature and E, F, G and H are independently selected from -CH-, $-CH_2-$, O, S, N, Se, Te, and $-NR_2-$, wherein $R_2$ is selected from hydrogen, alkyl moieties and functionalized alkyl moieties; and $R_1$ is selected from alkyl moieties and functionalized alkyl moieties. Polymers blended with or having the disclosed nonlinear optical materials as pendant side chains and exhibiting second order nonlinear optical properties are also disclosed.

The present invention relates to aromatic and heteroaromatic compounds with nonlinear optical (NLO) properties. In particular, the present invention relates to NLO compounds having high strength electron donating groups.

The compounds of the present invention, when suitably oriented, are capable of highly efficient second harmonic generation and electro-optic modulation of an electromagnetic wave having a wavelength between 300 nm and 2,000 nm. The present invention further relates to polymer blends of the disclosed compounds and to polymers having side chains of the disclosed compounds.

Highly efficient NLO materials capable of doubling or tripling the frequency of incident light are currently of great scientific and technological interest for use in optical telecommunications, signal processing and the construction of optical computers. Nonlinear optics is concerned with the interaction of electromagnetic fields in various media to produce new fields which may be altered in phase, frequency or amplitude. The NLO effect of a material upon an electromagnetic field is a function of the second and higher order terms of the following equation:

$$P = \alpha E + \beta E^2 + \alpha E^3 + \ldots$$

P is the polarization of a material, E is the intensity of the electric field, and the coefficients $\alpha$, $\beta$, $\gamma$, etc. are indicative of the NLO susceptibility of the material. Such coefficients are constant for a given material, but vary from material to material. The second order coefficient, $\beta$, for a given material, is indicative of the second harmonic generation properties of the material, with second harmonic generation efficiencies increasing as the value of $\beta$ increases.

Candidate NLO materials should possess good physical properties, such as high optical transparency, low dielectric constant, high laser damage threshold and good solubility in the solvents used for spin-casting of optical materials, and the like. The materials should also possess the mechanical and thermal properties required of NLO materials, in particular, high $\beta$ values, fast response times and nonlinear susceptibility over a broad range of wavelengths, particularly at wavelengths between about 300 nm and about 2,000 nm.

Recent efforts in the development of NLO materials have focused upon non-centrosymmetric organic materials with large delocalized pi-electron systems, which exhibit great nonlinear susceptibilities and can be varied to optimize the desired physical and mechanical properties. This includes the single benzene ring derivative disclosed by U.S. Patent No. 4,894,186 to Gordon and the compounds derived from two to four benzene rings separated by pi-electron conjugated carbon-carbon, carbon-nitrogen and nitrogen-nitrogen bridges disclosed by U.S. Patent No. 4,892,681 to Myata et al., U.S. Patent No. 4,894,263 to Dubois et al., U.S. Patent No. 4,933,112 to DeMartino et al. and U.S. Patent No. 4,935,292 to Marks et al. U.S. Patent No. 4,933,122 also discloses a two-ring compound containing a pyridine ring and a benzene ring separated by one of the above-listed pi-electron conjugated bridges.

Copending and commonly owned U.S. Patent Application Serial No. 07/626,358, filed December 12, 1990, discloses another group of NLO compounds containing from 2 to 10 six-membered aromatic or five-membered heteroaromatic rings or fused ring systems of such rings linked together by the above-listed pi-electron conjugated bridges. At least one five-membered heteroaromatic ring is present by itself, or as part of a fused ring system, which heteroaromatic ring contains at least one heteroatom selected from O, N, S or Se.

Still yet another group of NLO compounds is the group of compounds derived from highly conjugated fused ring structures of two or three aromatic or heteroaromatic rings, at least one of which is a five-membered heteroaromatic ring disclosed in copending and commonly owned U.S. Patent Application Serial No. 07/930,732, filed August 14, 1992, the disclosure of which is hereby incorporated herein by reference thereto. This application also discloses NLO compounds derived from one to four non-fused five-membered heteroaromatic rings linked together without pi-conjugated bridges.

To induce charge asymmetry, and consequently second order nonlinear polarizability, an aromatic or heteroaromatic ring at one end of the NLO compound structure is substituted with an electron donating group, while at the other end of the NLO compound structure an aromatic or heteroaromatic ring is substituted with an electron accepting group. The dipole of the compound structure can then be aligned in accordance with the method described by U.S. Patent No. 4,935,292.

The NLO compounds discussed above can be dissolved in a suitable polymer matrix, which is disclosed by the above-cited U.S. Patent Nos. 4,892,681 and 4,894,186. However, such combinations are often of limited solubility, resulting in crystallization of the guest NLO molecule out of the host matrix, or mobility of the guest molecules in the matrix, resulting in a loss of NLO performance.

The insolubility of these materials in a polymer matrix has been overcome by the covalent linking of the NLO compounds to polymer backbones. This is disclosed by the above cited U.S. Patent Nos. 4,894,263, 4,923,112 and 4,935,292. These references disclose polymers having NLO side chains covalently attached to the polymer by a reactive binding, typically a long chain alkyl group. The polymer is then spin-cast to form a film, which is then heated to near its glass-rubber transition temperature ($T_g$) to enhance molecular motion, including rotation of the NLO side chains. An intense electric field is then applied to the heated film for a given length of time and the film is then cooled to well below the $T_g$ in the presence of the electric field. This results in alignment of the dipoles of the side chains, providing a system in which the NLO components are locked in a preferred orientation while at the same time covalently linked within a polymer matrix.

According to U.S. Patent No. 4,935,292, NLO efficiency can be increased by repeatedly heating the material above and then cooling it below the $T_g$ several times prior to applying the electric field. It is disclosed that this reduces the number of pinholes, voids, free volume and other anomalies that can cause short circuits during poling, and also removes residual stress from the film.

U.S. Patent No. 4,894,263 discloses vinyl-type polymers linked to NLO side chains via an alkyl linker having up to 15 carbon atoms or a linker of the formula $(-CH_2-CH_2-O)_n-$, wherein n is between one and five. The linker is attached to the electron donating group of the NLO compound, which is selected from secondary amines, tertiary amines, and ether oxygen or a thioether sulphur. U.S. Patent No. 4,933,112 similarly discloses vinyl polymers linked to NLO side chains via electron donating group attached linkers. The electron donating groups are selected from secondary amines, an ether oxygen and a thioether sulphur.

U.S. Patent No. 4,935,292 discloses vinylic polymers such as polystyrene linked to NLO side chains via thallium-mediated esterification or by quaternization. The polymer is then joined to an alcohol substituent of the NLO compound structure or a pyridine ring within the structure.

The electron donating groups disclosed by these patents linking the NLO side chains to vinylic polymers are marginally electron donating in character and do not optimize the second order nonlinearity of the compounds disclosed. Stronger electron donating substituents are known; however, their introduction into NLO compounds, and the attachment of these compounds as polymer side chains, or even the simple blending of these compounds into polymer matrices, is problematic.

U.S. Patent No. 4,859,876 discloses an electron donating group having the structure:

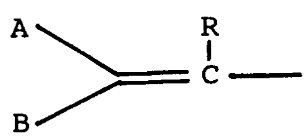

in which A and B are members of a five- to eight-member ring that is electron donating in nature and either unsaturated, partially saturated, or saturated, wherein A and B are selected from O, N, S, Se, Te and carbon, provided that both A and B are not carbon. R is hydrogen or an alkyl group. The example given of this electron donating group has the following structure:

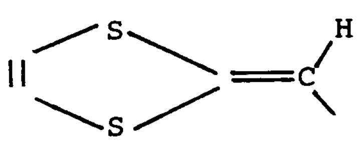

For this donor group in which R is hydrogen, synthesis of the NLO compound is difficult because of side reactions that occur at this double bond when introducing electron-accepting groups. The solubility of the resulting NLO compound in polymeric matrices and spin-casting solvents also decreases significantly. When R is alkyl, the solubility of the compound improves, but the reactivity of the ring double bond still interferes with introduction of electron accepting groups. The double bonds also interfere with the attachment of the NLO compounds as polymer side chains via the donor group.

Above-cited U.S. Patent Application Serial Nos. 07/626,358 and 07/930,732 disclose an electron donating group having the structure:

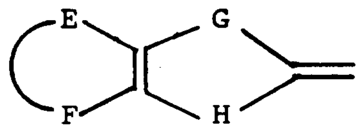

wherein E, F, G and H are members of a two-ring system having five- to eight-membered rings that are electron donating in nature. E, F, G and H are heteroatoms independently selected from O, N, S, Se and Te. This donating group also has limited solubility in polymeric matrices and spin-casting solvents, as well as a reactive vinyl double bond that interferes with the introduction of electron accepting groups to the NLO compound and attachment of the NLO compound as a polymer side chain via the donor group.

There remains a need for stronger electron donating groups that do not interfere with the introduction of electron accepting groups to NLO compounds or the attachment of the NLO compounds as polymer side chains via the donor groups.

It has now been discovered that substitution of reactive unsubstituted double bonds on certain strong electron donating groups of NLO compounds will reduce the reactivity of these double bonds without detracting from optical nonlinearity. Undesirable side reactions that occur when introducing electron accepting groups on the NLO compounds or when attaching the NLO compounds as polymer side chains via the donor groups are also eliminated. The substituent not only increases the solubility of the NLO compound in polymer matrices and spin-casting solvents, it also unexpectedly enhances the nonlinearity of the resulting NLO compound. Functionalizing the substituent will provide a means for grafting the NLO compounds onto polymers as side chains, and further enhances the nonlinearity of the NLO compounds.

Therefore, in accordance with one embodiment of the present invention, there is provided a nonlinear optical compound corresponding to Formula I:

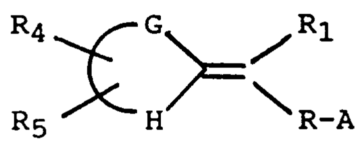

(I)

Wherein A is a first electron accepting group;

R is a pi-conjugated non-centrosymmetric organic moiety;

$R_4$ and $R_5$ are independently selected from hydrogen, alkyl moieties containing up to 12 carbon atoms and functionalized alkyl moieties containing up to 12 carbon atoms, wherein the functionalized alkyl moieties are selected from alkoxy, aminoalkyl, alkylhalide, hydroxyalkyl, alkylsulfide, alkylthiol, alkylazide, alkylcarboxylic, alkylsulfonic, alkylalkene and alkylalkyne moieties;

$R_1$ is selected from alkyl moieties containing up to 12 carbon atoms and functionalized alkyl moieties containing up to 12 carbon atoms, wherein the functionalized alkyl moieties are selected from alkyl, amino alkyl, alkylhalide, hydroxyalkyl, alkylsulfide, alkylthiol, alkylazide, alkylcarboxylic, alkylsulfonic, alkylalkene and alkylalkyne moieties; with the proviso that when $R_4$ and $R_5$ are hydrogen, $R_1$ contains at least three carbon atoms;

G and H are members of a saturated or unsaturated five- to eight-membered ring that is electron donating in nature, wherein G and H are independently selected from -CH-, $-CH_2-$, O, S, N, Se, Te and $-NR_2-$, wherein $R_2$ is selected from hydrogen, alkyl moieties containing up to 18 carbon atoms and functionalized alkyl moieties containing up to 18 carbon atoms, wherein the functionalized alkyl moieties are selected from alkoxy, aminoalkyl, alkylhalide, hydroxyalkyl, alkylsulfide, alkylthiol, alkylazide, alkylcarboxylic, alkylsulfonic, alkylalkene and alkylalkyne moieties;

wherein A, R, $R_1$, $R_4$, $R_5$ and said ring formed by G and H have a delocalized resonance configuration.

R can be any pi-conjugated non-centrosymmetric organic moiety exhibiting optical nonlinearity. R may contain from 1 to 10 aromatic or heteroaromatic rings or fused ring systems, linked together so as to form a delocalized resonance configuration. Suitable linkages include pi-electron conjugated carbon-carbon, carbon-nitrogen and nitrogen-nitrogen bridges. At least one five or six-membered heteroaromatic ring is preferably present, alone, or as part of a fused ring system, which heteroaromatic ring contains at least one heteroatom selected from O, N, S or Se. The number or size of the fused ring systems should not be so

large as to interfere with the solubility of the NLO compounds in polymer matrices or spin-casting solvents.

When $R_4$ and $R_5$ are hydrogen, the introduction of an alkyl or functionalized alkyl moiety having three or more carbon atoms at $R_1$ solubilizes the NLO compound in polymeric matrices and spin-casting solvents and increases the optical nonlinearity of the compound. $R_1$ is preferably a functionalized alkyl moiety, so that means are provided for grafting the NLO compounds onto polymers as side chains. The introduction of a functionalized alkyl moiety at $R_1$ also maximizes the enhancement of nonlinearity. Preferably, at least one of $R_4$ or $R_5$ is an alkyl or functionalized alkyl moiety, so that the reactivity of the ring double bond is reduced and the optical nonlinearity of the compound is increased. More preferably, at least one of $R_4$ or $R_5$ is a functionalized alkyl moiety, so that means are provided for grafting the NLO compounds onto polymers as side chains. When $R_4$ or $R_5$ is substituted with an alkyl or functionalized alkyl moiety, preferably at least one of $R_4$, $R_5$ or $R_1$ contains three or more carbon atoms to solubilize the NLO compound in polymer matrices and spin-casting solvents. When the ring is unsaturated, $R_4$ or $R_5$ is preferably substituted so that the reactivity of an unsaturated bond is reduced.

In accordance with another embodiment of the present invention, there is provided a nonlinear optical compound corresponding to Formula II:

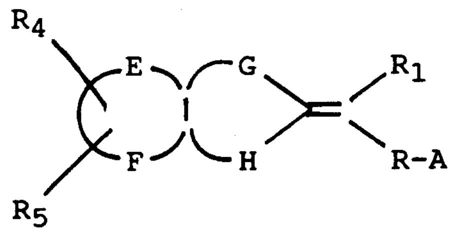

(II)

Wherein A is a first electron accepting group;

R is a pi-conjugated non-centrosymmetric organic moiety;

E and F, together with G and H, are members of a saturated or unsaturated two-ring system having five- to ten-membered rings that are electron donating in nature, wherein E, F, G and H are independently selected from -CH-, $-CH_2-$, O, N, S, Se, Te and $-NR_2-$, wherein $R_2$ is selected from hydrogen, alkyl moieties containing up to 18 carbon atoms and functionalized alkyl moieties containing up to 18 carbon atoms, wherein the functionalized alkyl moieties are selected from alkoxy, aminoalkyl, alkylhalide, hydroxyalkyl, alkylsulfide, alkylthiol, alkylazide, alkylcarboxylic, alkylsulfonic, alkylalkene and alkylalkyne moieties.

$R_1$ is selected from alkyl moieties containing up to 12 carbon atoms and functionalized alkyl moieties containing up to 12 carbon atoms, wherein the functionalized alkyl moieties are selected from alkoxy, aminoalkyl, alkylhalide, hydroxyalkyl, alkylsulfide, alkylthiol, alkylazide, alkylcarboxylic, alkylsulfonic, alkylalkene and alkylalkyne moieties;

$R_4$ and $R_5$ are independently selected from hydrogen, alkyl moieties containing up to 12 carbon atoms and functionalized alkyl moieties containing up to 12 carbon atoms, wherein the functionalized alkyl moieties are selected from alkoxy, aminoalkyl, alkylhalide, hydroxyalkyl, alkylsulfide, alkylthiol, alkylazide, alkylcarboxylic, alkylsulfonic, alkylalkene and alkylalkyne moieties;

The preferred species of R are the same as described above with respect to Formula I. The introduction of an $R_1$ alkyl or functionalized alkyl moiety eliminates the reactivity of the vinyl double bond and also increases the optical nonlinearity of the compound. $R_1$ is preferably a functionalized alkyl moiety, so that means are provided for grafting the NLO compounds onto polymers as side chains. The introduction of a functionalized alkyl moiety at $R_1$ also maximizes the enhancement of nonlinearity. More preferably, the $R_1$ alkyl or functionalized alkyl moiety contains three or more carbon atoms so that the NLO compound is soluble in polymeric matrices and spin-casting solvents.

Preferably, at least one of $R_4$ or $R_5$ is an alkyl or functionalized alkyl moiety to increase the solubility of the NLO compound in polymeric matrices and spin-casting solvents and to increase the optical nonlinearity of the compound. $R_4$ and $R_5$, and the preferred species thereof, are the same as described above with respect to Formula I.

Another embodiment of the present invention provides a combination exhibiting second order NLO properties. This combination includes a chemically inert medium and the NLO compounds of the present invention. The NLO compounds of these combinations preferably have an external field-induced molecular alignment.

In one aspect of this embodiment of the invention, the NLO compound is disposed as a layer or layers on a substrate of the chemically inert medium, which chemically inert medium is selected from glass, silica,

silicon and polymeric materials. In another embodiment of this aspect of the invention, the NLO compound is in the form of a blend of a guest compound in a host matrix, with the NLO compound of the present invention serving as the guest compound and the chemically inert medium serving as the host matrix. The chemically inert medium is preferably a thermoplastic polymer selected from polyacrylates, polymethacrylates, polyacrylamides, polycarbonates, polyamides, polyesters, polystyrenes, polyimides, polyether ketones, polyether etherketones, polyphenylene ethers and copolymers thereof.

In still another aspect of this embodiment of the present invention, pendant side chains of the NLO compounds of the present invention are covalently bonded to a chemically inert polymer material. In accordance with this aspect of the invention, $R_1$, $R_4$ or $R_5$ of the compound of Formula I or Formula II is a functionalized alkyl moiety, and the functionalized alkyl moiety is covalently linked to a polymer. The polymer contains one or more monomeric subunits having a reactive group capable of being covalently attached to a functionalized alkyl moiety, whereby the compound of Formula I or Formula II is covalently linked to the polymer at the reactive group of one of the subunits via the functionalized alkyl moiety.

Preferably, the polymer contains a plurality of the monomeric subunits having the reactive groups covalently substituted with an NLO compound via a functionalized alkyl moiety, so that the ratio of the monomeric subunits having reactive groups covalently linked to an NLO compound to monomeric subunits without an NLO compound covalently linked thereto is between 1:99 and about 50:50. The monomeric subunits having a reactive group capable of being covalently attached to a functionalized alkyl moiety and the monomeric subunits without an NLO compound covalently linked thereto are independently selected from polyacrylate, polyimide, polyamide, polyacrylamide, polystyrene, polyvinyl halide, polyacrylonitrile, polyvinyl alcohol, polyvinyl acetate, polyester, polyethylene, polypropylene, polyisobutylene, polyisoprene, poly(acid anhydride) and polycarbonate monomeric subunits.

The NLO compounds of the present invention, including the polymeric materials of the present invention, compared to the prior art, possess improved second order NLO properties, and increased solubility in polymer matrices and spin-casting solvents, attributable to substitution of the donor portion of the NLO compound structure with alkyl and functionalized alkyl moieties. In addition to possessing good second order NLO susceptibilities, good solubility and thermal and photochemical stability, the compounds of the present invention have high laser damage thresholds, are easily synthesized and have well-known and understood chemical properties.

The NLO compounds of the present invention, once suitably oriented, exhibit a high second order NLO susceptibility. NLO materials in accordance with the present invention contain NLO compounds having the structure of either Formula I or Formula II in which R can be any pi-conjugated non-centrosymmetric organic moiety exhibiting optical nonlinearity. Suitable non-centrosymmetric organic moieties contain from one to ten aromatic rings or fused ring systems. Two or more rings or fused ring systems are linked together to form a delocalized resonance configuration. Within the present specification "heteroaromatic" rings are defined as being limited to aromatic heterocyclic rings, thereby excluding carbocyclic rings such as phenyl groups. "Aromatic" rings are defined as generically including carbocyclic and heterocyclic rings. The heteroaromatic rings of the present invention contain one or more heteroatoms selected from O, N, S, Se and Te.

The non-centrosymmetric organic moieties of the present invention preferably contain from one to four aromatic rings or fused ring systems. The aromatic rings or fused ring systems within each non-centrosymmetric organic moiety may be the same or different.

For non-centrosymmetric organic moieties containing multiple rings or fused ring systems, it is preferable that at least one ring alone, or within a fused ring system, be a five-membered heteroaromatic ring having one heteroatom selected from O, N, S, Se and Te. The heteroaromatic ring may optionally include up to three additional N atoms. Preferably, the five-membered heteroaromatic rings possess a structure corresponding to Formula III:

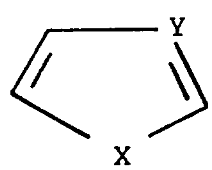

(III)

in which Y is C or N and X is selected from O, N, S, Se and Te.

Preferably, the non-centrosymmetric organic moieties containing multiple rings or fused ring systems contain two or more of the five-membered heteroaromatic rings, alone or as part of a fused ring system. Most preferably, all the rings in organic moieties containing multiple rings or fused ring systems are five-membered heteroaromatic rings, and all fused ring systems contain a five-membered heteroaromatic ring. When two or more heteroaromatic rings are present in a non-centrosymmetric organic moiety, the rings may have the same or different heteroatoms.

The fused ring systems should not be so large as to hinder the solubility of the NLO compounds in polymer matrices or spin-casting solvents. The point at which fused ring system size interferes with solubility is easily identified by those of ordinary skill in the art. Fused ring systems of two to three rings are preferred, and two ring systems are most preferred.

For non-centrosymmetric organic moieties containing multiple rings or fused ring systems, adjacent rings or fused ring systems are preferably linked by from one to three pi-electron conjugated functional groups such as carbon-carbon, carbon-nitrogen or nitrogen-nitrogen functional groups. Preferably, the adjacent rings or fused ring systems are bridged by one or two of the conjugated functional groups. When adjacent rings or fused ring systems are bridged by two or three functional groups, the conjugated functional groups may be the same or different and the number of conjugated functional groups between adjacent rings or fused ring systems may vary within an NLO compound. When the ring is heteroaromatic, or the fused ring system contains a heteroaromatic ring, the linkage is preferably substituted on the ring alpha to a heteroatom. For six-membered rings, alone, or within fused ring systems, the linkage is substituted para to another linkage, an electron donating group or an electron acceptor group.

The use of pi-electron conjugated functional groups to bridge adjacent rings or fused ring systems in NLO compounds is essentially conventional to the art of NLO active organic materials. Examples of suitable ring- or fused ring system-bridging functional groups known in the art include -CH=CH-, -N=N-, -CH=N-, -CH=N-N=CH-, -C≡C- and $(-CH=CH)_j$-, with j being from one to three.

The adjacent rings or fused ring systems of non-centrosymmetric organic moieties of linked aromatic rings or fused ring systems may also be linked by non-conjugated linkages. The adjacent rings or fused ring systems may also be covalently bonded between single ring members to directly link the rings or fused ring systems without forming a fused structure of the adjacent rings or fused ring systems.

The pi-conjugated non-centrosymmetric organic moieties of the present invention can also include a single aromatic ring or fused ring system. The preferred single ring is a five-membered heteroaromatic ring as defined above. Fused rings are preferred over single rings, and fused ring systems containing at least one five-membered heteroaromatic ring, as defined above, are preferred.

Regardless of whether the non-centrosymmetric organic moiety contains single or multiple rings or fused ring systems, the fused ring systems suitable for use in the present invention contain two or three rings. The two- or three-ring fused ring systems can consist entirely of five-membered heteroaromatic rings. A fused ring system consisting of three five-membered heteroaromatic rings is preferred over a system consisting of two five-membered heteroaromatic rings.

The configuration of multiple heteroaromatic rings within a fused ring system is not critical, and may be an all "up" configuration or an alternating "up" and "down" configuration, as depicted in the above-cited U.S. Patent Application Serial No. 930,732. The two or three five-membered heteroaromatic rings may have the same or different heteroatoms.

The fused ring systems of the present invention are not limited to structures containing only five-membered heteroaromatic rings. Two-ring fused ring systems suitable for use with the present invention may also contain a benzene or pyridine ring. Three-ring fused ring systems suitable for use with the present invention may contain up to two benzene or pyridine rings, or a benzene and a pyridine ring, in addition to the five-membered heteroaromatic ring. Thus, the fused ring systems of the present invention include such structures depicted in the above-mentioned U.S. Patent Application Serial No. 930,732. When a two- or three-ring system includes pyridine, the pyridine should not be quaternized. Such ionic species cause severe current leakage during the dipole-alignment electric field poling processes.

The fused ring compounds of the present invention are limited to two- and three-ring fused ring systems so as not to hinder the solubility of the NLO compounds in polymer matrices or spin-casting solvents. Three-ring fused ring systems are preferred because of their greater second order nonlinearity.

From the foregoing description, the aromatic and fused ring systems suitable for use with the present invention can be easily identified by those of ordinary skill in the art. Suitable rings and ring systems include, but are not limited to, pyrrole, furan, thiophene, imidazole, oxazole, thioazole, triazole, tetrazole, pyrazole, pyrimidine, purine, quinolines, carbazole, benzene, naphthalene, furazan, pyrazine, indole, isoindole, indazole, phenothiazine, benzotriazole, anthracene, phenanthrene, azophenanthrenes, quinazolines, pteridine and the like.

To induce charge asymmetry, the non-centrosymmetric organic moiety is substituted with an electron donating group and an electron accepting or withdrawing group. A first electron accepting group is depicted as A in Formula I and Formula II. The electron donating group and electron accepting group are substituted to the non-centrosymmetric organic moiety so as to form a delocalized resonance configuration. Positions for substituting electron donating and electron accepting groups to form a delocalized resonance configuration can be readily determined by those or ordinary skill in the art.

The electron donating and electron accepting groups are preferably substituted on five-membered heterocyclic rings that are either single rings or members of fused ring systems. More preferably, the electron donating and accepting groups are substituted alpha to a heteroatom of the five-membered heteroaromatic ring. For non-centrosymmetric organic moieties containing multiple rings or fused ring systems, the electron donating and electron accepting groups are preferably attached to aromatic or heteroaromatic rings or fused ring systems at opposite ends of the multiple ring structure.

For non-centrosymmetric organic moieties consisting of a single fused ring system, the electron donating and accepting groups are substituted to ring members of different rings so as to form a delocalized resonance configuration. Again, positions for substituting electron donating and electron accepting groups to form a delocalized resonance configuration can be readily determined by those of ordinary skill in the art.

The electron donating and electron accepting groups are preferably substituted on five-membered heterocyclic ring members of the fused ring systems, although this is not essential. For heteroaromatic rings, the electron donating group or electron accepting group is preferably substituted alpha to a heteroatom. Examples of typical delocalized resonance configurations are depicted in the above-cited U.S. Patent Application Serial No. 930,732.

The electron donating and accepting groups that are capable of inducing charge asymmetry to non-centrosymmetric organic moieties are essentially conventional to the art of NLO active organic materials. Any functional group capable of withdrawing electrons from a fused ring system is suitable for use as an electron accepting group.

Examples of suitable electron accepting groups known in the art include $-NO_2$, -CN, -CHO, $-COR_3$, $-COOR_3$, $-PO(OR_3)_2$, $-SO_2R_3$, $-SO_3R_3$, $-PO(R_3)_2$ and -CX=CYZ, wherein X, Y and Z are independently selected from hydrogen, -CN, $-NO_2$, $-COR_3$, $-COOR_3$, $-SO_2R_3$, $-PO(R_3)_2$ and $-PO(OR_3)_2$. $R_3$ is an alkyl group containing up to 15 carbon atoms, and preferably is a methyl group. Other suitable electron accepting groups include N,N-dialkylbarbituric acids, N,N-dialkylthiobarbituric acids, 3-dicyanovinylindane-1-sulfone, 1,3-bissulfonylindane, indane-1,3-dione, 3-dicyanovinylindane-1-one, 1,3-bisdicyanovinylindane and the electron accepting group having a structure corresponding to Formula IV:

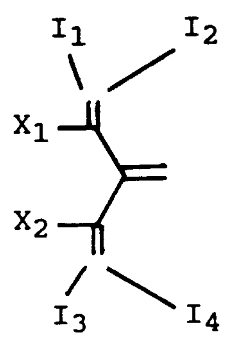

(IV)

wherein $I_1$, $I_2$, $I_3$ and $I_4$ are independently selected from cyano, nitro, ester, sulfonyl and phosphonyl groups, and $X_1$ and $X_2$ form a saturated or unsaturated five- to eight-membered cyclic ring or two ring system having five- to eight-membered rings.

Strong electron accepting groups are preferred, examples of which include $-C(CN)=C(CN)_2$, $-NO_2$, dicyanoethylene, dinitroethylene, cyanonitroethylene, nitroesterethylene, N,N-dialkylbarbituric acids, N,N-dialkylthiobarbituric acids, 3-dicyanovinylindane-1-sulfone, 1,3-bissulfonyl-indane, indane-1,3-dione, 3-dicyanovinylindane-1-one, 1,3-bisdicyanovinylindane and the group having the structure depicted in Formula IV, wherein $I_1$, $I_2$, $I_3$, $I_4$, $X_1$ and $X_2$ are the same as described above with respect to this structure. The most preferred strong electron accepting group is $-C(CN)=C(CN)_2$, a tricyanoethylene or tricyanovinyl group.

Any functional group capable of releasing electrons into the non-centrosymmetric organic moiety will function as an electron donating group. The improved electron donating groups disclosed by the present

invention are contained in the NLO compound structures depicted in Formula I and Formula II. The electron donating group portion of the NLO compound structure of Formula I has a structure corresponding to Formula V:

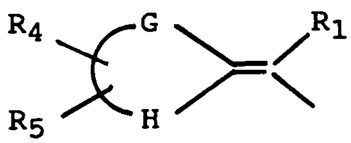

(V)

wherein $R_4$ and $R_5$ are independently selected from hydrogen, alkyl moieties containing up to 12 carbon atoms and functionalized alkyl moieties containing up to 12 carbon atoms, wherein the functionalized alkyl moieties are selected from alkoxy, aminoalkyl, alkylhalide, hydroxyalkyl, alkylsulfide, alkylthiol, alkylazide, alkylcarboxylic, alkylsulfonic, alkylalkene and alkylalkyne moieties;

$R_1$ is selected from alkyl moieties containing up to 12 carbon atoms and functionalized alkyl moieties containing up to 12 carbon atoms, wherein the functionalized alkyl moieties are selected from alkoxy, aminoalkyl, alkylhalide, hydroxyalkyl, alkylsulfide, alkylthiol, alkylazide, alkylcarboxylic, alkylsulfonic, alkylalkene and alkylalkyne moieties, with the proviso that when $R_4$ and $R_5$ are hydrogen, $R_1$ contains at least three carbon atoms.

$R_1$, $R_4$ and $R_5$ are more preferably functionalized alkyl moieties because such substituents not only increase the solubility of the resulting NLO compound in polymer matrices and spin-casting solvents, but also provide the greatest enhancement of the nonlinearity of the NLO compound and provide a means for grafting the NLO compound onto a polymer as a side chain. When the ring is unsaturated, $R_4$ and $R_5$ are preferably substituted so that the reactivity of an unsaturated bond is reduced.

G and H are members of a saturated or unsaturated five- to ten-membered ring that is electron donating in nature, wherein G and H are independently selected from -CH-, $-CH_2-$, O, N, S, Se, Te and $-NR_2-$, wherein $R_2$ is selected from hydrogen, alkyl moieties containing up to 18 carbon atoms, and functionalized alkyl moieties containing up to 18 carbon atoms, wherein the functionalized alkyl moieties are selected from alkoxy, aminoalkyl, alkylhalide, hydroxyalkyl, alkylsulfide, alkylthiol, alkylazide, alkylcarboxylic, alkylsulfonic, alkylalkene and alkylalkyne moieties. Preferably, G and H are independently selected from O, S, N, Se and Te. It is also preferred that G and H are members of a saturated ring. More preferably, the ring has a delocalized resonance configuration.

The electron donating group portion of the NLO compound structure of Formula II has a structure corresponding to Formula VI:

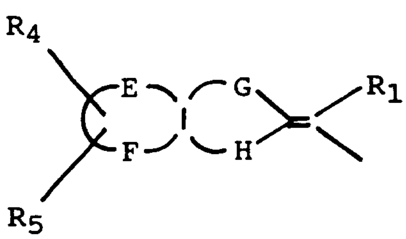

(VI)

wherein $R_1$ is selected from alkyl moieties containing up to 12 carbon atoms and functionalized alkyl moieties containing up to 12 carbon atoms, wherein the functionalized alkyl moieties are selected from alkoxy, aminoalkyl, alkylhalide, hydroxyalkyl, alkylsulfide, alkylthiol, alkylazide, alkylcarboxylic, alkylsulfonic, alkylalkene and alkylalkyne moieties. Again, $R_1$ is preferably a functionalized alkyl moiety..

E and F, together with G and H, are members of a saturated or unsaturated two-ring system having five- to ten-membered rings that are electron donating in nature, wherein E, F, G and H are independently selected from O, N, S, Se, Te, -CH-, $-CH_2-$ and $-NR_2-$, wherein $R_2$ is selected from hydrogen, alkyl moieties containing up to 18 carbon atoms, and functionalized alkyl moieties containing up to 18 carbon atoms, wherein the functionalized alkyl moieties are selected from alkoxy, aminoalkyl, alkylhalide, hydroxyalkyl, alkylsulfide, alkylthiol, alkylazide, alkylcarboxylic, alkylsulfonic, alkylalkene and alkylalkyne. Again, E, F, G and H are preferably independently selected from O, S, N, Se and Te. Preferably, the ring is unsaturated,

and more preferably, the ring has a delocalized resonance configuration.

$R_4$ and $R_5$, and the preferred species thereof, are the same as described above with respect to Formula V.

A preferred embodiment of the present invention includes a second electron donating group, or a second electron accepting group, or both, attached to the same rings, or ring members of fused ring systems, as the respective first electron donating group and the first electron accepting group, so that all of the electron donating and electron accepting groups present, together with the non-centrosymmetric organic moiety, form a delocalized resonance configuration. The second electron donating or accepting group may be the same or different than the corresponding first electron donating or accepting group. The inclusion of a second electron donating or electron accepting group increases the second order NLO properties of the resulting material as compared to materials having single-substitution of electron donating and electron accepting groups.

Examples of suitable second electron donating moieties well-known in the art for use in combination with the improved electron donating moieties of the present invention include $-NR_6R_7$, $-OR_8$, $-SR_8$, $-TeR_8$, $-SeR_8$, $-CH=NR_9$, $-CH=N-NH_2$, $-CH=N-N(R_6R_7)$ and $-CH=C[N(R_6R_7)]_2$, wherein $R_9$ is hydrogen or an alkyl moiety containing up to 10 carbon atoms, $R_8$ is an alkyl moiety containing up to 6 carbon atoms and $R_6$ and $R_7$ are independently selected from hydrogen, alkyl moieties containing up to 12 carbon atoms and functionalized alkyl moieties containing up to 12 carbon atoms, wherein the functionalized alkyl moieties are selected from alkoxy, aminoalkyl, alkylhalide, hydroxyalkyl, alkylsulfide, alkylthiol, alkylazide, alkylcarboxylic, alkylsulfonic, alkylalkene and alkylalkyne, or $R_6$ and $R_7$ together form a cyclic group containing up to 8 carbon atoms, including groups such as pyrrolidine, piperidine, piperazine and morpholine. Preferably, $R_8$ is a methyl group, $R_9$ is either hydrogen or a methyl group, and $R_6$ and $R_7$ are independently selected from methyl, ethyl, hexyl, cyclopentyl, cyclohexyl, pyrrolidine, piperidine, piperazine and morpholine.

The non-centrosymmetric organic moieties of the present invention may optionally be further substituted. Any number of functional groups can be substituted on the rings or fused ring systems of the non-centrosymmetric moieties, provided that the groups are not so large or so numerous to cause undesirable steric hindrance effects, the occurrence of which will be clear to those of ordinary skill in the art.

For example, the non-centrosymmetric organic moieties of the present invention can be functionalized with appropriate substituents to increase the solubility of the materials both in polymer matrices and in spin-casting solvents. Ordinarily, the solubility would decrease as the number of rings or fused ring systems in the non-centrosymmetric organic moiety increases. Substituents that will increase the solubility of the non-centrosymmetric organic moieties are well-known to those of ordinary skill in the art. Preferred substituents include alkyl moieties containing between 3 and 12 carbon atoms and functionalized alkyl moieties containing between 3 and 12 carbon atoms, wherein the functionalized alkyl moieties are selected from alkoxy, aminoalkyl, alkylhalide, hydroxyalkyl, alkylsulfide, alkylthiol, alkylazide, alkylcarboxylic, alkylsulfonic, alkylalkene and alkylalkyne moieties.

The non-centrosymmetric organic moieties upon which the NLO compounds of the present invention are based are prepared by well-known methods widely reported in the prior art. The preparation of many of these moieties is disclosed in the above-cited U.S. Patent Application Serial Nos. 626,358 and 930,732. Some of the non-centrosymmetric organic moieties are commercially available. The electron accepting groups and electron donating groups can be substituted to the non-centrosymmetric organic moiety using conventional methods.

A general procedure for the preparation of pi-conjugated non-centrosymmetric organic moieties substituted with the electron donor groups of the present invention is illustrated by the following scheme. A non-centrosymmetric organic moiety is reacted with an alkyl acyl chloride as shown below to form a ketone:

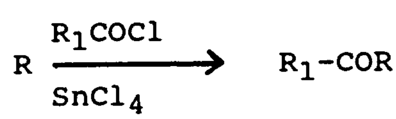


R and $R_1$ and the preferred species thereof are the same as described above with respect to Formula I and Formula II. The ketone is then reacted with a phosphonate ester of the desired electron donating group. For purposes of illustration, the electron donating group of Formula I is shown:

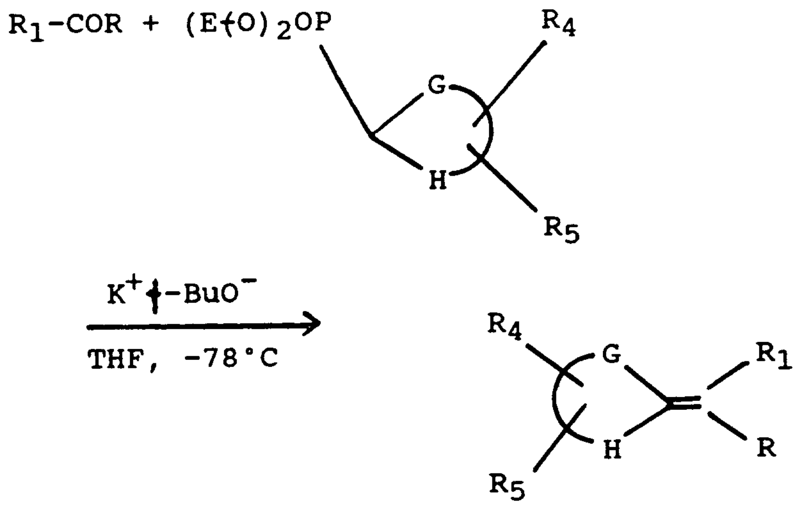


The phosphonate ester is formed as described in Tet. Lett., 41, 3695 (1976). $R_4$, $R_5$, G and H, and the preferred species thereof, are the same as described above, with respect to Formula I. The electron donating groups of Formula II may be similarly reacted.

The resulting intermediate product may be reacted by well-known methods to attach an electron accepting group to R. For example, if R contains a heteroaromatic ring, alone or as part of a fused ring system, for attachment of an electron accepting group, then the intermediate product can be reacted with tetracyanoethylene in DMF at about 0°C to tricyanovinylate the intermediate as follows:

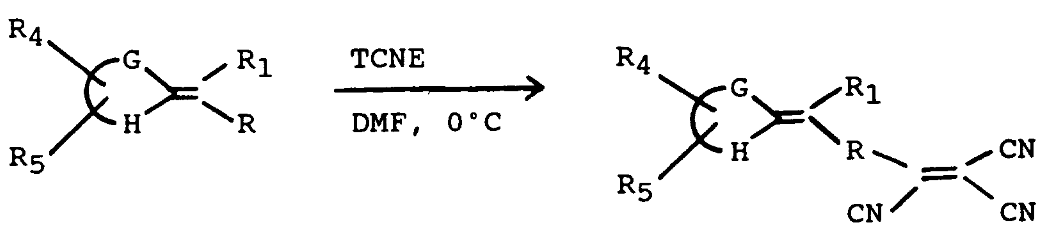


Other electron accepting groups may be attached as described in the above-cited prior art literature.

The NLO compounds of the present invention can be formed into a nonlinear optical material by combining the NLO compounds with a chemically inert medium. For example, the NLO compounds can be layered on a substrate such as glass, silica or polymeric materials, as described in U.S. Patent No. 4,894,186 to Gordon, the disclosure of which is hereby incorporated herein by reference thereto.

In another embodiment, a nonlinear optical medium can be formed by blending the NLO compounds of the present invention with a host thermoplastic polymer. Suitable host thermoplastic polymers include polyacrylates, polymethacrylates, polyacrylamides, polyimides, polycarbonates, polyesters, polyamides, polystyrenes and the like. This combination is also described in U.S. Patent No. 4,894,186, the disclosure of which is also hereby incorporated herein by reference thereto.

The NLO compounds of the present invention can also be covalently attached as side chains to the monomeric subunits of polymers. Polymers should be chosen for use with the present invention having monomeric subunits that have reactive functional groups for attachment of the side chains.

The polymer should have excellent optical transparency, good film-forming characteristics, a low dielectric constant and a relatively high $T_g$ for stable dipole orientation of the side chains. Other properties will come into consideration, depending upon the particular end-use requirements of the material; however, these properties are well-understood by those of ordinary skill in the art.

One class of polymers suitable for use with the present invention are polymers and copolymers, the monomeric subunits of which are derived from vinyl monomers such as acrylates, acrylamides, vinyl halides, acrylonitriles, ethylene, propylene, isobutylene, isoprene, acid anhydrides, styrenes, vinyl alcohols and vinyl acetates. Examples of other polymers suitable for use with the present invention include

polyimides, polyamides, polycarbonates, polyesters, polyphenylene ethers, polyetherimides, polyetherketones and polyether etherketones.

The polyacrylates suitable for use with the present invention include alkyl branched polyacrylates such as polymethacrylates. Likewise, the polyacrylamides suitable for use with the present invention include alkyl branched polyacrylamides such as polymethacrylamide, and the polyacrylonitriles include alkyl branched polyacrylonitriles such as polymethacrylonitrile.

Those of ordinary skill in the art are capable of identifying the functional groups of the polyacrylates, polyimides, polyamides, polyacrylamides, polyvinylhalides, polyacrylonitriles, polyvinylalcohols, polyvinylacetates, polyesters, polyphenylene ethers, polyetherimides, polyether ketones, polyether etherketones, poly(acid anhydrides) and polycarbonates to which the NLO compounds of the present invention can be attached to form side chains by conventional addition and condensation reactions.

Although the monomeric side units of polystyrene, polyethylene, polypropylene, polyisobutylene and polyisoprene do not have such functional groups, such monomeric subunits can first be functionalized to form a reactive group for the attachment of the NLO compound, such as the chloromethylation of polystyrene and the subsequent conversion to the more reactive iodomethyl derivative set forth in U.S. Patent No. 4,935,292 to Marks, the disclosure of which is herein incorporated by reference thereto. Alternatively, a functionalized derivative of these polymers can be used as a starting material, such as the poly(p-hydroxystyrene), the use of which is also disclosed by U.S. Patent No. 4,935,292.

For attachment of the NLO compounds to the monomeric subunits, $R_1$, $R_4$ or $R_5$ of the NLO compound of Formula I or Formula II is a functionalized alkyl moiety. The functionalized alkyl moiety is covalently linked to the polymer at a reactive functional group of one of the monomeric subunits suitable for attachment of the NLO side chains. Thus, the NLO compounds are covalently linked to a polymer at the reactive groups of monomeric subunits via functionalized alkyl moieties on the electron donor groups of the NLO compounds.

For NLO compounds covalently linked to polymers as pendant side chains, the electron accepting groups, non-centrosymmetric organic moieties, $R_1$, $R_2$, $R_4$, $R_5$, E, F, G, H and the preferred species thereof are the same as described above with respect to Formula I and Formula II. Polymers in accordance with the present invention will not be completely substituted with NLO side chains. The present invention includes polymers having ratios of NLO substituted monomeric subunits to unsubstituted monomeric subunits between about 1:99 and about 50:50. Substitution ratios between about 5:95 and about 40:60 are preferred. Substitution ratios less than about 30:70 are more preferred in order that the polymer remains soluble in solvents utilized in the preparation of NLO materials. The most preferred substitution ratio is about 25:75.

In preferred NLO polymers, the monomeric subunits are independently selected from monomers of ethylene, acrylates, alkyl-branched acrylates, acrylamides, alkyl-branched acrylamides, styrenes, $\alpha$-alkylstyrenes, vinyl acetate, ether ketones and ether etherketones. When one of the monomeric subunits is a styrene monomer, the aromatic ring may be further substituted by one or more hydroxyl or alkyl groups, provided that the groups are not so large or so numerous to cause undesirable steric hindrance effects, the occurrence of which will be clear to those of ordinary skill in the art. In the most preferred NLO polymers, the monomeric subunits are independently selected from monomers of acrylates, methacrylates, ether ketones and ether etherketones.

The polymerization of the polymeric NLO materials of the present invention is essentially conventional and is readily understood by those of ordinary skill in the art. Depending on the material in question, in some cases, it is preferable first to polymerize the polymer and then attach the side chains to the reactive groups of the monomeric subunits. In other cases, it is preferable to synthesize a monomer having an NLO side chain covalently attached thereto to be copolymerized with a monomer having no side chain.

In either case, a preferred method for preparation of the polymeric NLO materials of the present invention, however, first synthesizes either a polymer or a monomer having a pre-NLO side chain covalently attached thereto. The pre-NLO side chain is defined as an NLO side chain having no electron accepting group. Once the pre-NLO polymer is formed, either by attaching pre-NLO side chains to an existing polymer or by copolymerizing monomers having pre-NLO side chains with a monomer having no NLO or pre-NLO side chain, the resulting pre-NLO polymer can be reacted to attach electron accepting groups to the pre-NLO side chains to provide a polymeric NLO material.

The synthesis of polymers having pre-NLO side chains is essentially conventional and well-understood by those of ordinary skill in the art, as is the synthesis of monomers having pre-NLO side chains and the copolymerization of same with a monomer having no NLO or pre-NLO side chains. The preferred electron accepting group is tetracyanoethylene, which tricyanovinylates the pre-NLO side chains. The pre-NLO polymer can be reacted with tetracyanoethylene in a basic solvent at an elevated temperature to achieve such tricyanovinylation.

For polymers having monomeric subunits such as acid anhydrides, epoxides, acid halides, isocyanates, carboxylic acids, esters, sulfonic acids and amines, the pre-NLO side chain can be directly attached to the polymer, rather than first synthesizing a monomer having a pre-NLO side chain that is then copolymerized with an unsubstituted monomer to form a pre-NLO polymer. For example, pre-NLO side chains can be reacted with poly(styrene maleic anhydride) to form a poly(styrene maleimide) having pre-NLO side chains. The method by which pre-NLO side chains can be directly attached to polymers is essentially conventional and well understood by those of ordinary skill in the art. The polymer having pre-NLO side chains can then be reacted to attach electron accepting groups such as tricyanovinyl groups to the pre-NLO side chains to provide a polymeric NLO material.

For the tricyanovinylation of pre-NLO polymers, suitable basic solvents for the reaction of tetracyanoethylene and the pre-NLO polymer include N,N-dimethylformamide (DMF), pyridine, N,N-dimethylacetamide, N-methylpyrrolidone, tertiary amines and the like. The preferred solvent is DMF. A reaction mixture is prepared by dissolving the polymer and the tetracyanoethylene in one or more of the above solvents. The reaction mixture is heated to a temperature between about 50 °C and about 140 °C, preferably to about 100 °C, to obtain the tricyanovinylated polymers.

The degree of tricyanovinylation of the polymer is limited only by the number of pendant pre-NLO groups available for tricyanovinylation. Therefore, a slight equivalent excess of the tetracyanoethylene over the pre-NLO polymer should be used.

As noted above, the reaction of the polymer and the tetracyanoethylene can be carried out at temperatures in the range of from about 50 °C to about 140 °C. Higher temperatures will result in an increased rate of reaction, and even higher rates can be achieved by pressurizing the reaction vessel to elevate the boiling point of the solvent, allowing the reaction to proceed at an even higher temperature. However, a reaction temperature of 100 °C is preferred to minimize inter- and intra-molecular cross reactions.

To insure uniform mixing of the polymer and the tetracyanoethylene, the reaction mixture should be maintained at a constant state of mild agitation. It is also preferred that the reaction mixture be maintained under an atmosphere of an inert gas.

Once the reaction is complete, the NLO polymer is precipitated with a lower alkyl alcohol, such as methanol or isopropanol, filtered and dried under vacuum. The polymer can then be further purified by conventional methods, typically by repeated dissolution and reprecipitation from the lower alkyl alcohol.

A general procedure employed to synthesize acrylate monomers with pre-NLO side chains, followed by copolymerization with an unsubstituted acrylate monomer, and activation of the pre-NLO side chains is illustrated below, wherein R, $R_4$, $R_5$, G and H, and the preferred species thereof, are the same as described above with respect to Formula I.

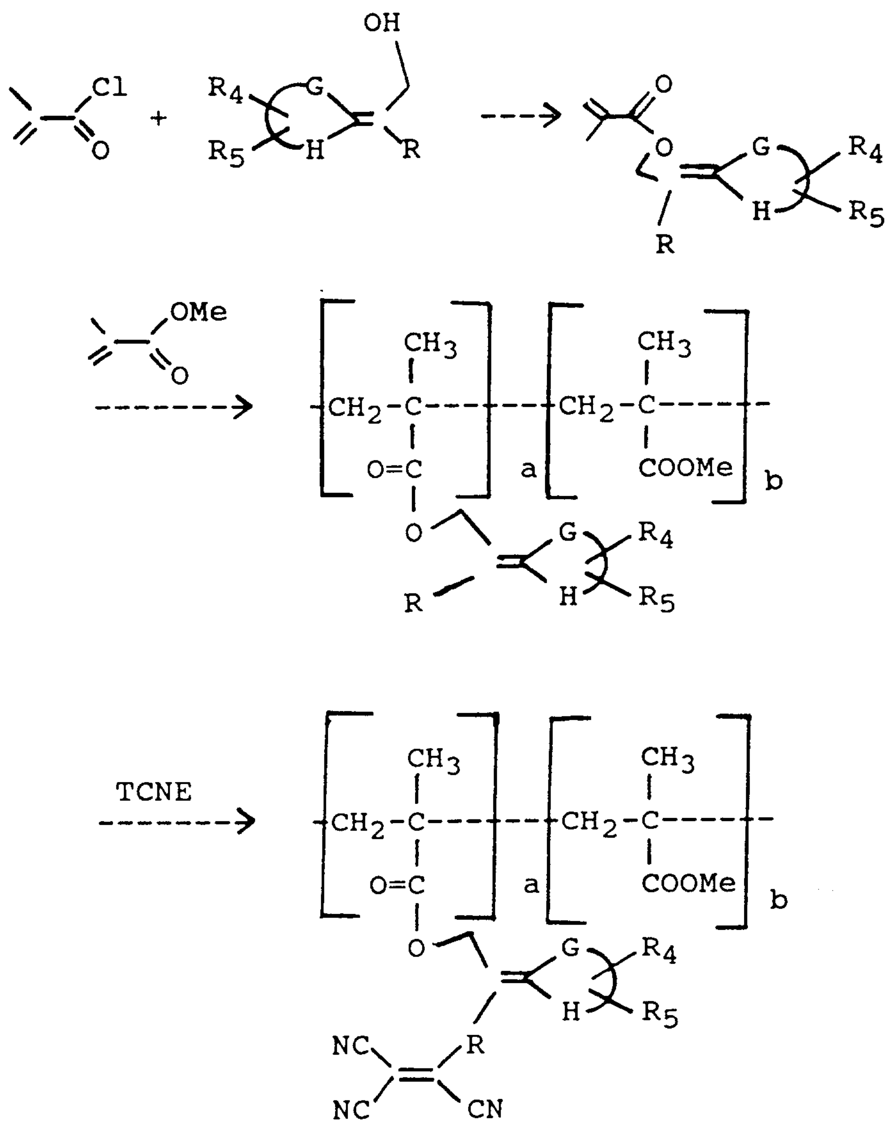


The NLO polymers are recovered and purified by conventional means known to those of ordinary skill in the art. Films of the polymers may be formed by spin-coating, after which the films may be repetitively annealed prior to poling at an elevated temperature near the $T_g$ of the material. Following annealing, the dipoles of the side chains may be aligned by application of an intense electric field (0.2-1.0 MV $cm^{-1}$) at temperatures near the $T_g$. The foregoing sequence of spin-coating, annealing and poling is essentially conventional and disclosed in U.S. Patent No. 4,935,292, the disclosure of which is hereby incorporated herein by reference thereto.

It is disclosed in U.S. Patent No. 4,935,292 and SPIE proceeding No. 1147, 74-83 (1989) that further stabilization of the NLO side chain alignment can be achieved by a radiation-induced or chemical-induced cross-linking of the polymer matrix. This process is also essentially conventional, and the disclosure of which in U.S. Patent No. 4,935,292 is also hereby incorporated herein by reference thereto.

The preferred pre-NLO and NLO polymers of the present invention typically have weight-average molecular weights between about 5,000 and about 300,000 daltons measured by GPC or light scattering. The incorporation of tricyanovinyl groups increases the $T_g$'sof the pre-NLO polymers.

The electro-optic coefficient of an NLO-active poled polymer film is proportional to the product of the molecular second order nonlinear optical susceptibility coefficient, $\beta$, and the molecular ground state electric dipole moment, $\mu$. The molecular $\beta$ is dependant upon the frequency at which the measurement is performed because of the resonance effect near the absorption peak. A method to compare molecules with different absorption properties by extrapolation of the $\beta$ value measured at a specific frequency to zero frequency using a two-level model is disclosed by Singer, J. Opt. Soc. Am., B6, 1339-50 (1989). The $\beta$

value at the extrapolated zero frequency is defined $\beta_0$. The NLO-active molecules of the present invention can exhibit values of the $\beta_0\mu$ product as high as about 9,000 in units of $10^{-48}$ esu measured at a wave length of 1,907 nm.

Thus, it can be appreciated that the present invention provides NLO compounds combining second order nonlinear optical properties with the physical, mechanical and optical properties required of an optical material, together with thermal and photochemical stability. The following examples are further illustrative of the present invention, and are not to be construed as limiting the scope thereof. Unless otherwise indicated, materials were obtained from Aldrich Chemical Supply. All parts and percentages are by weight unless expressly indicated to be otherwise and all temperatures are in degrees Celsius.

## EXAMPLES

### EXAMPLES 1-10 - Synthesis Of Acyl Thiophenes

Acyl thiophenes were prepared by reacting thiophene, thienylthiophene, dithienylthiophene and 2,2'-bisthiophene with acetyl chloride, hexanoyl chloride and nonanoyl chloride. Each sample was prepared following the procedure set forth below for thienylthiophene and hexanoyl chloride.

### EXAMPLE 1 - Preparation Of Hexanoyl Thienylthiophene

Thienylthiophene (1.5 g, 10.7 mmol) and hexanoyl chloride (1.52 g, 11.3 mmol), both of which were obtained from Aldrich and used without further purification, were dissolved in benzene (20 mL) and cooled to 0°C under argon. Stannic Chloride (10.7 mL, 10.7 mmol, 1 M solution in dichloromethane) was added dropwise and the resulting solution was stirred overnight. (Upon addition of the Stannic Chloride, the color changed from colorless to either red or green.) After stirring overnight, 20 mL of a 50:50 mixture of concentrated hydrochloric acid and water was added. The resulting mixture was stirred for two hours whereupon the color became golden yellow. The organic layer was separated, washed with sodium bicarbonate solution, dried ($Na_2SO_4$) and concentrated to yield 2-hexanoyl thienylthiophene (1.81 g, 93% yield). The compound was further purified by column chromatography using hexane/dichloromethane as the elutant.

### EXAMPLE 2

2-Nonanoyl thienylthiophene was prepared as in Example 1, substituting nonanoyl chloride (1.99 g, 11.3 mmol) for hexanoyl chloride. An 85% yield was obtained.

### EXAMPLE 3

2-Acetyl thiophene was prepared as in Example 1, substituting thiophene (0.9 g, 10.7 mmol) for thienylthiophene and acetyl chloride (0.89 g, 11.3 mmol) for hexanoyl chloride. A 96% yield was obtained.

### EXAMPLE 4

2-Hexanoyl thiophene was prepared as in Example 1, substituting thiophene (0.9 g, 10.7 mmol) for thienylthiophene. A 94% yield was obtained.

### EXAMPLE 5

2-Acetyl dithienylthiophene was prepared as in Example 1, substituting dithenylthiophene (1.5 g, 10.7 mmol) for thienylthiophene and acetyl chloride (0.89 g, 11.3 mmol) for hexanoyl chloride. An 84% yield was obtained.

### EXAMPLE 6

2-Hexanoyl dithienylthiophene was prepared as in Example 1, substituting dithenylthiophene (1.5 g, 10.7 mmol) for thienylthiophene. An 85% yield was obtained.

EXAMPLE 7

2-Nonanoyl dithienylthiophene was prepared as in Example 1, substituting dithienylthiophene (1.5 g, 10.7 mmol) for thienylthiophene and nonanoyl chloride (2.0 g, 11.3 mmol) for hexanoyl chloride. A 91% yield was obtained.

EXAMPLE 8

5-Acetyl (2,2'-bisthiophene) was prepared as in Example 1, substituting 2,2'-bisthiophene (1.66 g, 10.0 mmol) for thienylthiophene and acetyl chloride (0.86 g, 11.0 mmol) for hexanoyl chloride. A yield of 93% was obtained.

EXAMPLE 9

5-Hexanoyl (2,2'-bisthiophene) was prepared as in Example 1, substituting 2,2'-bisthiophene (1.78 g, 10.7 mmol) for thienylthiophene. A yield of 91% was obtained.

EXAMPLE 10

5-Nonanoyl (2,2'-bisthiophene) was prepared as in Example 1, substituting 2,2'-bisthiophene (2.0 g, 12.0 mmol) for thienylthiophene and nonanoyl chloride (2.25 g, 12.7 mmol) for hexanoyl chloride. A yield of 89% was obtained.

EXAMPLES 11-14

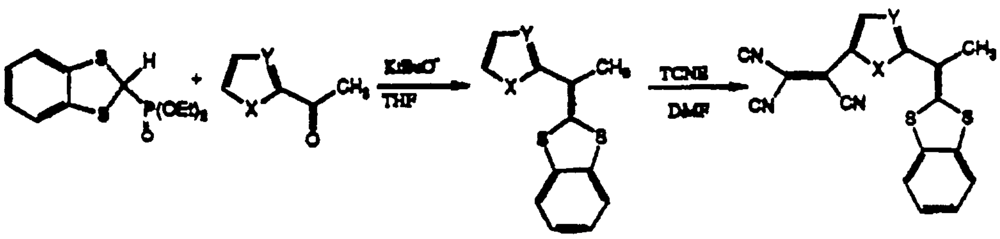


| EXAMPLE | X | Y |
|---|---|---|
| 11 | S | CH |
| 12 | O | CH |
| 13 | S | N |
| 14 | N-$CH_3$ | CH |

EXAMPLE 11

2-Ethylthiophene-6-ylidene-[2'-(1',3'benzodithiole)] was prepared by adding potassium t-butoxide (1.12 g, 10.0 mmol) to a -78°C stirred solution of 2-acetyl thiophene (1.26 g, 10.0 mmol) and 2-diethoxy phosphoryl-1,3-benzodithiole (2.9 g, 10.0 mmol), in dry, freshly distilled, tetrahydrofuran (25 mL) under an argon atmosphere. The 2-acetyl thiophene was obtained from Aldrich and used without further purification, although the material prepared in Example 3 would have also been suitable. The 2-diethoxy phosphory-1,3-benzodithiole was prepared as described in J. Org. Chem., 39, 2457 (1974). The resulting solution was stirred and slowly warmed to room temperature overnight under an argon atmosphere. The mixture was concentrated in vacuo, and the residue was dissolved in dichloromethane, washed with water (3 x 200 mL), dried ($Na_2SO_4$), and concentrated onto approximately 5.0 g silica. This silica was added to a medium

pressure column packed with silica. The column was eluted with a 3:1 ratio solution of hexane to dichloromethane with a gradient to 1:1 to yield the substituted thiophene (2.36 g, 90% yield).

Tricyanovinylation of the substituted thiophene was accomplished by gradually adding (approx. 5-10 min.) tetracyanoethylene (1.0 equiv.) to a stirred solution of the substituted thiophene in 25 mL dimethylformamide (DMF) at 0 °C under an argon atmosphere. The reaction mixture was slowly warmed to room temperature overnight. The reaction mixture was poured into 100 mL of water and extracted with dichloromethane (2 x 200 mL). The dichloromethane layers were combined, dried ($Na_2SO_4$), concentrated in vacuo and chromatographed (silica) on a medium pressure column. The column was eluted with a 2:1 ratio of hexane to dichloromethane to yield the tricyano compound (3.1 g, 95% yield).

EXAMPLE 12

2-Ethylfurano-6-ylidene-[2'-(1',3'-benzodithiole)] was prepared as in Example 11, by substituting 2-acetyl furan for the 2-acetyl thiophene. The 2-acetyl furan was obtained from Aldrich and used without further purification. An 87% yield of the substituted furan was obtained. The substituted furan was tricyanovinylated according to the procedure of Example 11 (85% yield).

EXAMPLE 13

2-Ethylthiazolo-6-ylidene-[2'-(1',3'-benzodithiole)] was prepared as in Example 11, by substituting 2-acetyl thiazole for the 2-acetyl thiophene. The 2-acetyl thiazole was obtained from Aldrich and used without further purification. An 88% yield of the substituted thiazole was obtained. The substituted thiazole was tricyanovinylated according to the procedure of Example 11 (80% yield).

EXAMPLE 14

N-Methyl-2-ethylpyrrolo-6-ylidene-[2'-(1',3'-benzodithiole)] was prepared as in Example 11 by substituting 2-acetyl N-methyl pyrrole for the 2-acetyl thiophene. The 2-acetyl N-methyl pyrrole was obtained from Aldrich and used without further purification. An 85% yield of the substituted (N-methyl pyrrole) was obtained. The substituted (N-methyl pyrrole) was tricyanovinylated according to the procedure of Example 11 (60% yield).

EXAMPLES 15-17

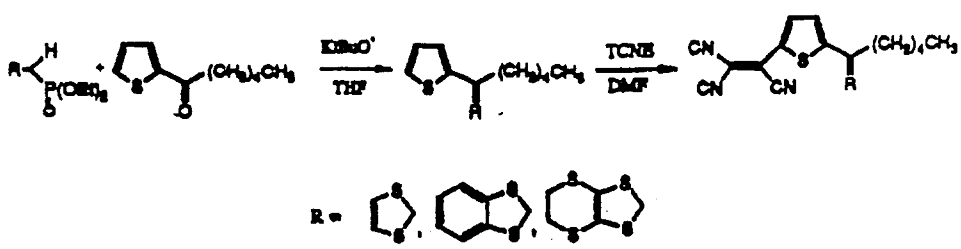


15 16 17

EXAMPLE 15

2-Hexyl-thienyl-6-ylidene-[2'-(1',3'-benzodithiole)] was prepared by reacting the 2-hexanoyl thiophene of Example 4 with 2-diethoxy phosphoryl-1,3-dithiole following the procedure of Example 11. The 1,3-dithiolium phosphonate ester was prepared as described in Tet. Lett., 41, 3695 (1976). The substituted thiophene was tricyanovinylated according to the procedure of Example 11 (80% yield).

EXAMPLE 16

2-Hexyl-thienyl-6-ylidene-[2'-(1',3'-benzodithiole)] was prepared according to Example 15 by substituting the 2-diethoxy phosphoryl-1,3-benzodithiole of Example 11 for the 1,3-dithiolium phosphonate ester. A

yield of 83% was obtained. This product was tricyanovinylated according to the procedure of Example 11 (95% yield).

EXAMPLE 17

2-Hexyl-thienyl-6-ylidene-[2'-(5',6'-dihydro-1',3'-dithiolo [4,5-b] [1,4] dithiin was prepared according to Example 15 by substituting 2-diethoxy phosphory-(ethylenedithio-1,3-dithiole) for the 1,3-dithiolium phosphonate ester. The ethylene dithio-1,3-dithiolium phosphonate ester was prepared as described in Synthesis, 26, 24 (1991). A 74% yield was obtained. This product was tricyanovinylated according to the procedure of Example 11 (50% yield).

EXAMPLES 18-20

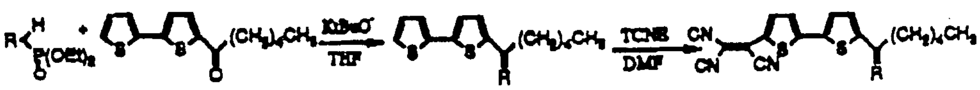


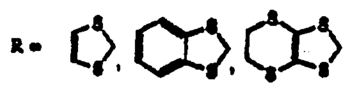

18 19 20

In Examples 18-20, the 5-hexanoyl (2,2'-bisthiophene) of Example 9 was substituted for the 2-hexanoyl thiophene of Examples 15-17 and was reacted with the respective phosphonate esters. Yields of 80%, 92% and 75%, respectively, were obtained. The compounds were tricyanovinylated according to the procedure of Example 11 and yields of 30%, 45% and 25% were obtained.

EXAMPLES 21-23

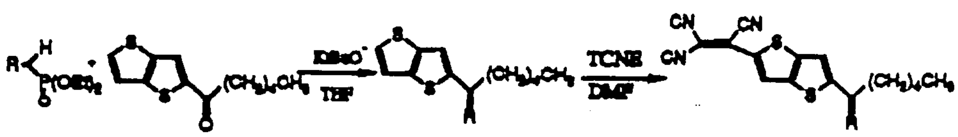


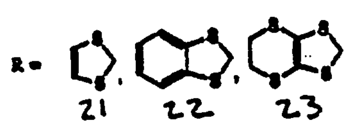

21 22 23

In Examples 21-23, the 2-hexanoyl thienylthiophene of Example 1 was substituted for the 2-hexanoyl thiophene of Examples 15-17 and was reacted with the respective phosphonate esters. Yields of 81%, 87% and 72%, respectively, were obtained. The compounds were tricyanovinylated according to the procedure of Example 11 and yields of 20%, 35% and 25%, respectively, were obtained.

EXAMPLES 24-27

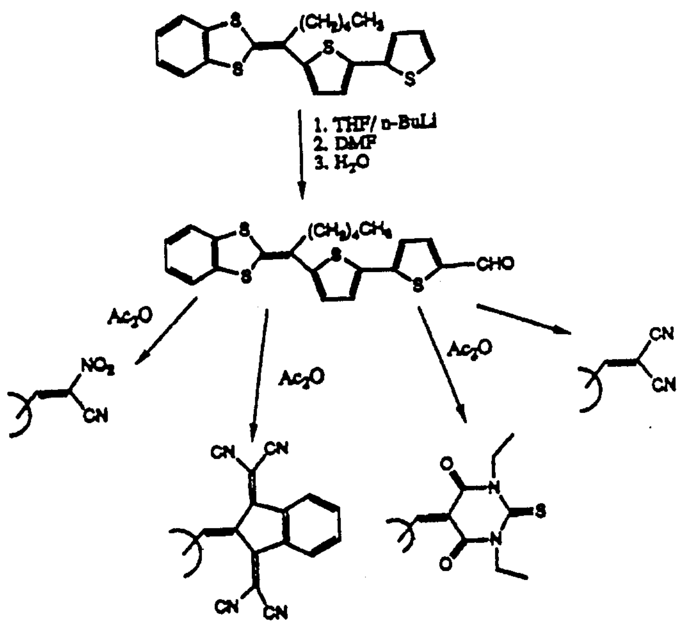


To a stirred solution of the 5-hexyl-(2,2'-bisthienyl)-6-ylidene-[2"-(1",3"-benzodithiole)] of Example 19 (4.06 g, 10 mmol) in THF (25 mL) at 0°C was added n-butyllithium (2.5 M, 4.2 mL, 10.5 mmol). The mixture was allowed to warm to room temperature under an argon atmosphere over 30 minutes. The resulting mixture was stirred for one hour and then cooled back to 0°C at which time DMF (5 mL, excess) was added. The solution was warmed to room temperature and stirred under an argon atmosphere for two hours. The reaction was then quenched with 10 mL water. The THF was removed in vacuo and the resulting oil was chromatographed with a 3:1 ratio of hexane to dichloromethane to yield the 5-substituted 5'-formyl (2,2'-bisthiophene) (3.2 g, 75% yield) as an orange solid.

This aldehyde was then reacted with a variety of electron acceptors in the following manner. 1,3-Diethyl-2-thiobarbituric acid (1.00 g, 5.00 mmol), nitroacetonitrile (0.43 g, 5.0 mmol), malononitrile (0.33 g, 5.0 mmol) or tetracyanoindane (1.21 g, 5.0 mmol) was dissolved in acetic anhydride (5-10 mL) and warmed to approximately 60°C for 30-60 minutes under an argon atmosphere. The 1,3-diethyl-2-thiobarbituric acid and malononitrile were obtained from Aldrich and used without further purification. The nitroacetonitrile was prepared as described in Izv. Akad. Nauk SSR, Ser. Khim., 10, 2342 (1986). The tetracyanoindane was prepared as described in J. Chem. Soc. Perkins Trans., 11, 865 (1987). At this time, the aldehyde (2.16 9, 5.0 mmol) was added and the mixture was maintained at 60°C for 1-2 hours. The mixture was cooled, poured into 200 mL water and extracted with dichloromethane (3 x 100 mL). The dichloromethane fractions were combined, dried ($Na_2SO_4$), concentrated and chromatographed with successive 4:1, 2:1 and 1:1 ratio solutions of hexane to dichloromethane to yield the desired product. A yield of 80% was obtained for the nitroacetonitrile derivative. A yield of 75% was obtained for the tetracyanoindane derivative. A yield of 80% was obtained for the 1,3-diethyl-2-thiobarbituric acid derivative. A yield of 90% was obtained for the malononitrile derivative.

Comparative Example 1

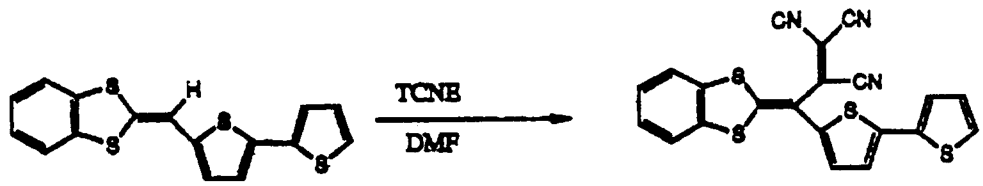


A mixture of 5-formyl (2,2'-bisthiophene) (1.95 g, 10.0 mmol) and the 2-diethoxy phosphory-1,3-benzodithiole of Example 12 (2.40 g, 10.0 mmol) were cooled to -78 °C in THF (25 mL) under an argon atmosphere. Potassium t-butoxide (1.12 g, 10.0 mmol) was added and the mixture was stirred overnight and allowed to warm to room temperature. The THF was removed in vacuo and the resulting oil was redissolved in 100 mL dichloromethane. The dichloromethane was washed with water (2 x 75 mL), dried ($Na_2SO_4$) and concentrated onto 2.5 g silica gel. The mixture was placed onto a medium pressure column and flashed with a gradient of hexane/dichloromethane ranging from 5/1 to 1/1 to yield 5-methyl-(2,2'-bisthienyl)-6-ylidene-[2"-(1",3"-benzodithiole)] (2.15 g, 65% yield).

The resulting compound was tricyanovinylated by gradually adding (approx. 5-10 min.) tetracyanoethylene (1.28 g, 10.0 mmol) to a stirred solution of it (3.30 g, 10.0 mmol) in 40 mL DMF at 0 °C under an argon atmosphere. The reaction mixture was slowly warmed to room temperature overnight. The resulting mixture was poured into 300 mL of water and extracted with dichloromethane (2 x 150 mL). The dichloromethane layers were combined, dried and concentrated as described above. The mixture was placed onto a medium pressure column and flashed with a gradient of a 3:1 ratio solution of hexane and dichloromethane. The sole reaction product was the undesired 6-substituted tricyanovinyl derivative (2.81 g, 65% yield).

Comparative Example 2

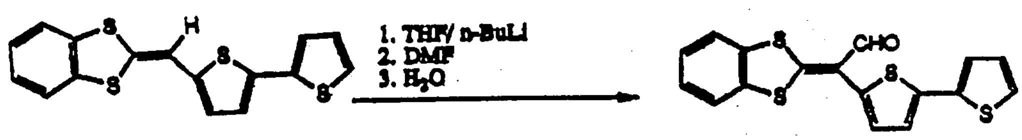


5-Methyl-[2,2'-bithienyl]-6-ylidene[2"-(1",3"-benzodithiole)] was prepared as described in Comparative Example 1. A quantity of this material (3.30 g, 10.0 mmol) was dissolved in dry THF (50 mL) at 0 °C, and purged with argon. To this solution was added n-butyllithium (2.5 M, 4.2 mL, 10.5 mmol) and the solution was allowed to warm to room temperature under an argon atmosphere over 30 minutes. The resulting mixture was stirred for one hour and then cooled back to 0 °C at which time DMF (5 mL, excess) was added. The solution was warmed to room temperature and stirred under an argon atmosphere for two hours. The reaction was then quenched by the addition of 10 mL water. The THF was removed in vacuo and flash chromatographed as in Comparative Example 1 to yield the undesired 6-substituted compound as the only product (2.51 g, 70% yield).

The foregoing examples illustrate the advantage obtained by blocking the vinyl hydrogen of the electron donating groups of the present invention to prevent undesirable side-reactions. The side products typically possess significantly reduced NLO activity. Alkyl substitution of this vinyl group effectively blocks the side reactions without affecting NLO activity. In fact, the second order nonlinearity increases with alkyl substitution of the vinyl group.

The NLO compounds of the present invention thus possess a combination of second order nonlinearity and reaction yield heretofore unobtained by the prior art. These compounds also possess the thermal stability and photochemical stability required of NLO materials. At the same time, the compounds have good solubility, high laser damage thresholds, are easily synthesized and have well-known and understood chemical properties. NLO compounds with the electron donating groups of the present invention represent a

versatile family of compounds that can be readily varied to increase their second order NLO properties.

The foregoing examples and description of the preferred embodiment should be taken as illustrating, rather than as limiting, the present invention as defined by the claims. As will be readily appreciated, numerous variations and combinations of the features set forth above can be utilized without departing from the present invention as set forth in the claims. Such variations are not regarded as a departure from the spirit and scope of the invention, and all such modifications are intended to be included within the scope of the following claims.

## Claims

**1.** A nonlinear optical compound of the general formula (I):

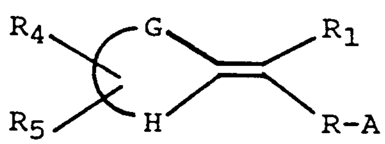

(I)

wherein A is a first electron accepting group;

R is pi-conjugated non-centrosymmetric organic moiety;

$R_4$ and $R_5$ are independently selected from hydrogen, alkyl moieties containing up to 12 carbon atoms and functionalized alkyl moieties containing up to 12 carbon atoms, wherein said functionalized alkyl moieties are selected from alkoxy, aminoalkyl, alkylhalide, hydroxyalkyl, alkylsulfide, alkylthiol, alkylazide, alkylcarboxylic, alkylsulfonic, alkylalkene and alkylalkyne moieties;

$R_1$ is selected from alkyl moieties containing up to 12 carbon atoms and functionalized alkyl moieties containing up to 12 carbon atoms, wherein said functionalized alkyl moieties are selected from alkyl, aminoalkyl, alkylhalide, hydroxyalkyl, alkylsulfide, alkylthiol, alkylazide, alkylcarboxylic, alkylsulfonic, alkylalkene and alkylalkyne moieties, with the proviso that when $R_4$ and $R_5$ are hydrogen, $R_1$ contains at least 3 carbon atoms; and

G and H are members of a saturated or unsaturated five- to ten-membered ring that is electron donating in nature, wherein G and H are independently selected from -CH-, $-CH_2-$, O, N, S, Se, Te, and $-NR_2-$, wherein $R_2$ is selected from hydrogen, alkyl moieties containing up to 18 carbon atoms and functionalized alkyl moieties containing up to 18 carbon atoms, wherein the functionalized alkyl moieties are selected from alkoxy, aminoalkyl, alkylhalide, hydroxyalkyl, alkylsulfide, alkylthiol, alkylazide, alkylcarboxylic, alkylsulfonic, alkylalkene and alkylalkyne moieties;

wherein A, R, $R_1$, $R_4$, $R_5$ and said ring formed by G and H comprise a delocalized resonance configuration;

or of the general formula (II):

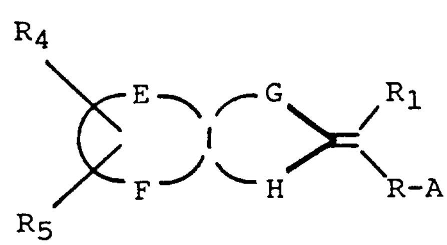

(II)

wherein A, R, $R_4$ and $R_5$ are as defined above;

$R_1$ is as defined above without the proviso;

E, F, G and H are members of a saturated or unsaturated two-ring system having five- to ten-membered rings that are electron donating in nature, wherein E, F, G and H are independently selected from -CH-, $-CH_2-$, O, N, S, Se, Te and $-NR_2-$, wherein $R_2$ is as defined above; and

wherein $R_1$, $R_4$, $R_5$ and said two-ring system formed by E, F, G and H comprise a delocalized resonance configuration.

2. The compound of claim 1, wherein R comprises from one to ten aromatic rings or fused ring systems.

3. The compound of claim 2, wherein R comprises at least one heteroaromatic ring, or at least one fused ring system comprising a heteroaromatic ring, said heteroaromatic ring preferably comprising a five-membered heteroaromatic ring, particularly a five-membered heteroaromatic ring having the structure:

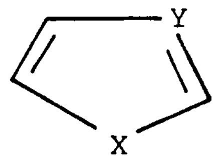

wherein Y is C or N and X is selected from O, S, Se, Te and N.

4. The compound of any one of claims 2 and 3, wherein all of said rings comprise said five-membered heteroaromatic ring, and all of said fused ring systems contain said five-membered heteroaromatic ring.

5. The compound of claim 2, wherein R comprises two or more rings or fused ring systems linked together to form a delocalized resonance configuration.

6. The compound of claim 5, wherein R comprises from two to four aromatic rings or fused ring systems.

7. The compound of any one of claims 5 and 6, wherein at least two adjacent rings or fused ring systems are linked together by a conjugated functional group comprising from one to three moieties independently selected from -CH = CH-, -N = N-, -CH = N-, -CH = N-N = CH-, -C≡C- and $(-CH = CH)_j$-, wherein j is from one to three.

8. The compound of any one of claims 5 and 6, wherein at least two adjacent rings or fused ring systems are linked together by at least one non-conjugated linkage.

9. The compound of any one of claims 5 and 6, wherein said two or more rings or fused ring systems comprise at least two adjacent rings or fused ring systems directly covalently bonded together without forming a fused structure of said adjacent pair.

10. The compound of any one of claims 2 to 9, wherein R comprises one or more aromatic rings independently selected from pyrrole, furan, thiophene, imidazole, oxazole, thiazole, triazole, tetrazole, pyrazole, pyrimidine, purine, quinolines, carbazole, benzene, naphthalene, furazan, pyrazine, indole, isoindole, indazole, phenothiazine, benzotriazole, anthracine, phenanthrene, quinazoline, pteridine and azophenanthrenes, particularly selected from pyrrole, furan, thiophene, thiazole and oxazole.

11. The compound of any one of claims 2 to 10, wherein R comprises at least one fused ring system containing two or three aromatic rings.

12. The compound of any one of claims 2 to 11, wherein R consists essentially of a single fused ring system containing two or three aromatic rings.

13. The compound of claim 12, wherein said fused ring system comprises at least one and preferably two or three five-membered heteroaromatic rings having the structure:

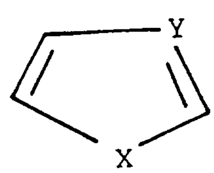

wherein Y is C or N and X is selected from O, S, Se and N.

**14.** The compound of any one of claims 1 to 13, wherein said electron accepting group is selected from $-NO_2$, -CN, -CHO, $-COR_3$, $-COOR_3$, $-PO(OR_3)_3$, $-SO_2R_3$, $-SO_3R_3$, $-PO(R_3)_2$ and -CX=CYZ, wherein X, Y and Z are independently selected from hydrogen, -CN, $-NO_2$, $-COR_3$, $-COOR_3$, $-SO_2R_3$, $-PO(R_3)_2$ and $-PO(OR_3)_2$, wherein $R_3$ is selected from alkyl moieties containing up to 15 carbon atoms; and preferably comprises tricyanoethylene.

**15.** The compound of any one of claims 1 to 13, wherein said electron accepting group is selected from N,N-dialkylbarbituric acids, N,N-dialkylthiobarbituric acids, indane-1,3-dione, 3-dicyanoindane-1-one, 1,3-bis-dicyanovinylindane, 3-dicyanovinylindane-1-sulfone and 1,3-bissulfonylindane.

**16.** The compound of any one of claims 1 to 13, wherein said electron accepting group comprises

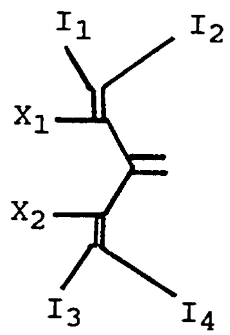

wherein $I_1$, $I_2$, $I_3$ and $I_4$ are independently selected from cyano, nitro, ester, sulfonyl and phosphonyl, and $X_1$ and $X_2$ form a saturated or unsaturated five- to eight-membered cyclic ring or two-ring system having five- to eight-membered rings.

**17.** The compound of any one of claims 1 to 16, further including a second electron accepting group attached to the same ring as said first electron accepting group, so that said delocalized resonance configuration is maintained.

**18.** The compound of any one of claims 1 to 17, wherein $R_1$, $R_4$ and $R_5$ are independently selected from alkyl moieties and functionalized alkyl moieties and preferably at least one of $R_1$, $R_4$ and $R_5$ comprises a functionalized alkyl moiety.

**19.** The compound of claim 1, wherein G and H and E, F, G and H, respectively, are independently selected from O, N, S, Se and Te.

**20.** The compound of any one of claims 1 to 19, wherein in the case of formula (I) G and H are members of an unsaturated ring and in the case of formula (II) E, F, G and H form a saturated ring.

**21.** The compound of any one of claims 1 to 20, further including a second electron donating group attached to R in the same manner as said first electron donating group so that said delocalized resonance configuration is maintained, wherein said second electron donating group is selected from $-NR_6R_7$, $-OR_8$, $-SR_8$, $-TeR_8$, $-SeR_8$, $-CH=NR_9$, $-CH=N-NH_2$, $-CH=N-N(R_6R_7)$ and $-CH=C[N(R_6R_7)]_2$, wherein $R_9$ is hydrogen or an alkyl moiety containing up to six carbon atoms, $R_8$ is an alkyl moiety containing up to six carbon atoms and $R_5$ and $R_6$ are independently selected from hydrogen, alkyl moieties containing up to 12 carbon atoms and functionalized alkyl moieties containing up to 12 carbon atoms wherein said functionalized alkyl moieties are selected from hydroxyl, ethylene, acetylene, amine, thiol, sulfonic acid and carboxylic acid, or $R_6$ and $R_7$ together form a cyclic moiety containing up to 8 carbon atoms.

**22.** The compound of claim 21, wherein said second electron donating group comprises said first electron donating group.

**23.** A combination exhibiting second order nonlinear optical properties comprising a chemically inert medium and said nonlinear optical compound of any one of claims 1 to 22.

**24.** The combination of claim 23, wherein said nonlinear optical compound has an external field-induced molecular alignment.

**25.** The combination of any one of claims 23 and 24, comprising a blend of said nonlinear optical compound in a chemically inert medium selected from polyacrylates, polymethacrylates, polyacrylamides, polycarbonates, polyamides, polyesters, polystyrenes, polyimides, polyether ketones, polyether etherketones, polyphenylene ethers and copolymers thereof.

**26.** The combination of any one of claims 23 to 25, wherein $R_1$, $R_4$ or $R_5$ comprises said functionalized alkyl moiety and said functionalized alkyl moiety is covalently linked to a polymer comprising one or more monomeric subunits having a reactive group capable of being covalently attached to a functionalized alkyl moiety, whereby said nonlinear optical compound is covalently linked to said polymer at said reactive group of one of said subunits via said functionalized alkyl moiety, said polymer preferably comprising a plurality of said monomeric subunits having reactive groups covalently substituted with a nonlinear optical compound via a functionalized alkyl moiety, so that the ratio of said monomeric subunits having reactive groups covalently linked to a nonlinear optical compound to monomeric subunits without a nonlinear optical compound covalently linked thereto is between about 1:99 and about 50:50, preferably between about 5:95 and about 40:60 and most preferably is about 25:75.

**27.** The combination of claim 26, wherein said monomeric subunits having reactive groups covalently substituted with a nonlinear optical compound via a functionalized alkyl moiety, and said monomeric subunits without a nonlinear optical compound covalently linked thereto are independently selected from acrylate, imide, amide, acrylamide, styrene, vinyl halide, acrylonitrile, vinyl alcohol, vinyl acetate, polyester, polyphenylene ether, polyether ketone, polyether etherketone, acid anhydride, ethylene, propylene, isobutylene, isoprene and polycarbonate monomeric subunits.

**28.** The combination of any one of claims 26 and 27, wherein said functionalized alkyl moiety comprises from four to six carbon atoms.

European Patent Office

# PARTIAL EUROPEAN SEARCH REPORT

which under Rule 45 of the European Patent Convention shall be considered, for the purposes of subsequent proceedings, as the European search report

Application Number EP 93 12 0340

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| D,A | EP-A-0 493 716 (ISTITUTO GUIDO DONEGANI)<br>* the whole document *<br>--- | 1-14, 19-28 | G02F1/35<br>C07D409/06<br>C07D409/14<br>C07D417/06<br>C07D495/04 |
| A | EP-A-0 357 783 (SUMITOMO ELECTRIC INDUSTRIES)<br>* claims *<br>--- | 1,18-20 | |
| A | EP-A-0 364 313 (RHONE-POULENC CHIMIE)<br>* the whole document *<br>--- | 1,14,19, 20,23-25 | |
| A | EP-A-0 422 900 (KABUSHIKI KAISHA)<br>* the whole document *<br>--- | 1-3,10 | |
| A | SPIE, ADVANCES IN NONLINEAR POLYMERS AND INORGANIC CRYSTALS, LIQUID CRYSTALS<br>vol. 824 , 1987<br>pages 86 - 92<br>H.E.KATZ, C.W.DIRK ET AL. 'exceptional second-order nonlinear optical susceptibilities in organic compounds'<br>* Table 1, Compounds No. 6,8 *<br>----- | 1,14,23 | TECHNICAL FIELDS SEARCHED (Int.Cl.5)<br>G02F |

## INCOMPLETE SEARCH

The Search Division considers that the present European patent application does not comply with the provisions of the European Patent Convention to such an extent that it is not possible to carry out a meaningful search into the state of the art on the basis of some of the claims
Claims searched completely :
Claims searched incompletely :
Claims not searched :
Reason for the limitation of the search:

see sheet C

| Place of search | Date of completion of the search | Examiner |
|---|---|---|
| THE HAGUE | 11 March 1994 | Puetz, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

INCOMPLETE SEARCH

Claims searched incompletely : 1-28

Reason : The scope of the claims would appear to be broader than is justified by the extent of the description (e.g. the examples); hence the claims are not considered to be fully supported by the description, contrary to the requirements of Article 84 EPC. Consequently, the search has been limited to only that subject-matter of the claims (rule 45 EPC) which is supported by the examples.